# EUROPEAN PATENT APPLICATION

(11) **EP 4 230 157 A1**
(43) Date of publication of application: **23.08.2023**
(21) Application number: 22157814.9
(22) Date of filing: 21.02.2022
(51) Int. Cl.: A61B 17/34, A61B 8/08, A61M 5/42

(54) **MOUNTABLE DEVICE FOR AUTONOMOUS BLOOD VESSEL INTERFACING AND INSTRUMENT ACTUATION**

(71) Applicant: Neurescue ApS, 1150 København K (DK)
(72) Inventor: Frost, Habib, 2300 København S (DK); Huldt, Olof, 21622 Limhamn (SE); Nørløv, Mads Bundgaard, 1169 København K (DK)
(74) Representative: Inspicos P/S

(57) **Abstract**

Disclosed is a mountable device for autonomous blood vessel mapping and instrument actuation configured to provide vascular access in an anatomical region selected from a group consisting of: Frontal part of the thigh, frontal part of the pelvis, frontal part of the groin, the wrist, frontal part of the arm and/or frontal part of the neck. The device is comprised of a housing or a frame (12), fixation structure, and a detection structure for detecting presence of a blood vessel, the detection structure defining a detection path along the skin surface. It is further comprising at least one sensor (1, 2) arranged to provide a signal indicative of the presence of a blood vessel along the detection path, a computing unit, and an interface, said interface configured to communicate the entry location to the user for manual insertion of the percutaneous needle and/or cannula or to communicate the entry location to a movement structure for manual or automatic insertion of the percutaneous needle and/or cannula.

## Description

### INTRODUCTION

The invention relates to a device for detection of blood vessels in anatomical regions of a human or animal. The invention further relates to a method of using the device.

### BACKGROUND

The success of delivering life-saving emergency interventional procedures is often inversely correlated with the time to carry out said interventional procedures. Emergency interventional procedures are often carried out in suboptimal conditions and often without operators optimally trained in the procedures and devices which are required by the emergency situation, thus increasing the complexity and probability of failure of the interventional procedures. It is in particularly limited by the availability of highly trained specialist physicians, e.g. interventional cardiologists or interventional radiologists, who are present at only selected and few highly specialized hospitals and who are not present in the pre-hospital emergency care arena.

The ability to stabilize patients using emergency interventional procedures relies on physicians' abilities to promptly gain access to the intended vessel, through which interventional procedure can be performed, prior adequate training and procedural volume experience to perform the procedure under the emergency duress and to perform the procedure under difficult clinically elective circumstances.

Alternatively, emergency blood transfusion or blood sample obtainment or other vascular procedures may be enabled by a vascular access procedure.

A delay in the emergency interventional procedure, caused by a delay in vessel access or the lack of relevant trained physicians in the critical emergency treatment window, may lead to permanent patient injury or death and leads to lowered procedure outcomes in the current state of the art (Romagnoli et al. J Trauma Acute Care Surg. 2017 Dec;83(6):1161-1164)

Many existing specialized hospital-based interventional procedures could profoundly improve the standard of care, if 1) made readily available to a wider range of hospitals and a wider range of physician operators and non-physician operators, i.e., acute care nurses and paramedics, 2) is made available in the emergency room setting, the pre-hospital setting and the. Said procedures include, but are not limited to, temporary aortic balloon occlusion, cardiopulmonary bypass, percutaneous ventricular assist devices, ECMO.

Temporary aortic occlusion is one example of a life-saving interventional procedure for the treatment of cardiac arrest and non-compressible haemorrhage. Cardiovascular disease contributes 30.9% of global mortality. Currently only 1 out of 10 survive a cardiac arrest to hospital discharge. It is responsible for higher mortality rates than any other disease in industrialized countries, and three-quarters of non-infectious mortality in developing countries. In the US there are around 350.000 cardiac arrests outside of hospitals; and at least as many inside hospitals. The potential for improvement is massive. In 2010, the cost of medical care for heart disease in the US was $273 billion and the loss in productivity was $172 billion.

A main hindrance in performing interventional procedures is gaining access to the intended blood vessel within the required therapeutic window, i.e., time frame. This limits the availability of life saving interventional treatments for patients, thus adversely affecting patient outcomes. Current state of the art in establishing emergency vascular access requires a very high amount of specialized clinical operator experience and thus limits the accessibility to this procedure, and said operators are limited to the fewest of hospitals.

The femoral artery is the most-used used access pathway for performing interventional cardiology and interventional radiology procedures, with a size facilitating inserting catheters such as for insertion of a percutaneous ventricular assist device or for extracorporeal membrane oxygenation (ECMO), both life-saving emergency procedures not available at most hospitals that do not have the subspecialists able to deliver these procedures. It is not possible to use techniques or devices configured for superficial vein access such as for e.g. veinous blood sample withdrawal or peripheral venous access sheaths, such as a Venflon^{™} device.

The femoral artery is a deeply located large artery and is the second-largest artery in the body after the aorta. The average depth from skin to the femoral artery in normal-weight persons is 1,5-3 cm. In obese persons it is 3-7 cm. The mean femoral artery diameter in adult men is estimated as 9,8 mm and in women is determined as 8,2 mm.

The femoral artery is significantly different than e.g. the cubital vein, an often-used access site for withdrawing venous blood samples. The cubital vein is a 1 mm diameter superficial vein with 0.5-1 mm distance from the skin to the blood vessel. A device such as a handheld vein detection system using illumination of the location of superficial veins on the surface of the skin is thus unfeasible in assisting a clinical operator in achieving femoral artery access.

The state of the art for detecting a large blood vessel, e.g. the femoral artery or the carotid artery, includes manually hand-manipulated ultrasound probes, i.e. ultrasound probes that are held and mobilized by a clinical operator, where the prerequisite training of the operator must include training in how to correctly position the probe on the right intended location on the skin, knowledge of the anatomical region, prior training sessions on how to perform ultrasound-guided manual hand-held needle puncture while simultaneously operating the ultrasound probe with the non-dominant hand, and preventing an unacceptable level of movement and shaking of the patient, who may be in distress or in an in extremis clinical condition, while continuing to correctly position the angle, orientation and other geometric features of said hand-held manually operated probe. Since the probe can be manipulated in any of the six degrees of freedom, it requires a person specifically trained and skilled in the art to use the device to find an intended blood vessel, which limits the life-saving minimally invasive interventional procedures to particularly interventional radiologists and interventional cardiologist, who are not present at the majority of hospitals, who are not present in the majority out-of-hospital emergency care units.

### OBJECTS

It is an object of embodiments of the invention to provide a device capable of gaining emergency blood vessel access in highly time critical emergency settings, and under difficult clinical circumstances in elective settings, where interventional procedures would enable the delivery of a life-saving minimally invasive treatment, but where the delivery of said blood vessel access is not possible today, either due to e.g. time constraints, lack of available of the correct type of highly trained specialty personnel, e.g. at most hospitals and in the out-of-hospital emergency care setting, manual procedural difficulties in performing the procedure by the operator, and more.

Particularly, it is an object of the invention to enable the delivery of definitive treatments that are currently rendered impossible due to lack of adequate time, experience, and resources by the presently available medical personnel, who may not have the necessary volume of experience or procedure competency to perform the required vascular access procedure.

It is an object of embodiments to improve present hospital-based and out-of-hospital-based interventional procedures.

### SUMMARY

According to the above and other objects, the invention provides a device and a method according to the independent claims and with optional features according to the dependent claims.

In the following, the living being is referred to as a "patient". It could be a human being or an animal.

The blood vessel detection device comprises a housing or frame. The housing or frame defines a structure which can be attached to the body of the patient. A frame would be considered as an open structure which provides access to the skin of the patient through the open structure. A housing is a closed structure at most providing a specifically intended passage to the skin of the patient from within the housing. The housing may form an enclosing casing around the device on the skin of the patient.

The housing or frame can be attached to an anatomical region which means that device is configured to be positioned in a specific position and orientation such that the detection path intersects the blood vessel and thereby allows the detection structure to detect the blood vessel when sensing along the detection path across the blood vessel.

A fixation structure for supporting the housing or frame against the skin surface may be configured for fixation of the housing or frame to a skin surface. It could e.g. comprise an adhesive surface area of the housing or frame. The adhesive surface may be releasably attachable to the skin and ensure fixation and prevent sliding of the housing or frame relative to the skin and prevent accidental release of the housing or frame from the skin. The fixation structure could also comprise a belt or strap allowing the housing or frame to be strapped onto the patient, e.g. with a hook and loop fastener e.g. Velcro^{®}, a belt structure which can be fastened by traditional buckle clip or belt buckle, or the fixation structure could be a structure of the housing or frame which can be stitched directly to the skin of the patient. Alternatively, the fixation structure could comprise a back plate which a user can press against the skin surface of the patient. Such a back plate may particularly be shaped relative to the anatomical region,

The fixation structure is suitable for attachment to an anatomical region of the patient. The anatomical region is selected from a group consisting of: frontal part of a thigh, frontal part of a pelvis, frontal part of a groin, a wrist, a frontal part of an arm and a frontal part of a neck. The fixation structure may be configured, when attached to the anatomical region, to place the detection path of the detection structure transverse, non-parallel to a blood vessel.

For ensuring an intended position of the housing or frame relative to the anatomical region and thus relative to the intended blood vessel, the fixation structure may include a surface structure with a shape which matches the shape of the anatomical region in question. In one example, the surface structure is intended for continuous contact with the skin of the patient, e.g. with a forearm of the patient. For this purpose, the surface structure may have a curved shape, the width and length, and depth, and the specific curvature may be adapted to match the forearm. The surface structure may be curved, it may also include a relatively non-slippery material such as a soft cloth or rubber material preventing sliding relative to the skin, or an adhesive.

The surface structure may e.g. be shaped to facilitate that the device is in direct contact with the skin surface in an area of at least 10-100 square centimeter or more. This counteracts sliding of the device relative to the patient and thereby facilitates a more precise insertion of the needle or cannula into the entry location of the anatomical region.

The surface structure may have an elastically deformable structure. In this case, it may be a flat surface structure which can be bent to match a curved structure, e.g. a simple curvature (2D curvature) or a complex curvature (3D curvature) of the anatomical region. It may e.g. be constituted by a memory foam.

The fixation structure may include an expandable member, e.g. including a cuff which can be strapped onto the patient and inflated to match the curvature of the anatomical region and optionally exert pressure thereon. It could be inflated with a compressible or an incompressible medium, e.g. gas or liquid, e.g. atmospheric air, CO₂ ,water, or saline. Such an expandable member may be selectively activated or deactivated, e.g. controlled by an operator or automatically operated by the device.

The fixation structure may include a back element and a front element between which the anatomic region is compressed. The front and back elements could be releasable from each other and re-attachable to each other.

If the fixation structure includes belts or straps, they may be suitable for compressing the surface structure against the skin, e.g. such that the surface structure flattens the skin and the underlying tissues, e.g. connective tissue and fat.

If the fixation structure includes an adhesive, it may be suitable for flattening the skin and the underlying tissues, e.g. connective tissue and fat against the surface structure via an adhesive force.

The surface structure may be interchangeable such that different surface structures can be attached to the frame or housing and thereby adapt the fixation structure to different corresponding anatomical regions and/or sizes of the patient.

The detection structure is configured for detecting presence of the blood vessel, and comprises a sensor and a computing unit.

The sensor could be a transducer which herein is any component capable of converting one form of energy to another. Often this is from some detected energy into an electrical signal. This may be embodied by a piezoelectric component.

The sensor could be a 'probe'. Herein, the term "probe" is a housing containing one or more transducers. The probe may define a specific sensing direction, e.g. by forming a shielding structure allowing energy to be received from a specific direction and/or allowing energy to be emitted in a specific direction.

The computing unit may be configured to receive a signal from the sensor and therefrom determine the presence of the blood vessel and to define an insertion procedure for a needle and/or cannula.

The sensor could form a receiver for receiving a signal from the anatomical region, e.g. pressure waves or electromagnetic radiation. The sensor may e.g. have a structure comparable to a microphone. In this case, the sensor is configured to receive pressure waves arising as blood flows in the blood vessel. In this case, the computing unit is configured to identify the pressure wave being caused by blood flow in the blood vessel.

The sensor may alternatively be configured to receive pressure waves arising as an echo from a pressure wave transmitted into the anatomical region and returned by the body, e.g. from a blood vessel. This could e.g. be an ultrasound sensor or sound of other frequency. In this case, the computing unit may be configured not only to identify the pressure wave being reflected by a blood vessel, but may further be configured to identify a frequency of the received pressure wave and a shift in frequency relative to the transmitted pressure wave. This may be used by the computing unit to determine a doppler effect.

If the fixation structure is configured to compress the skin and underlying tissues, this may create a local compression of the intended blood vessel, and this may again create a turbulent flow which is more easily recognizable by the computing unit. The compression may be generated by said expandable member, thereby generating a turbulent flow selectively depending on activation or deactivation of the expandable member.

The detection structure may include a sensor in the form of an ultrasonic transducer or other means for detecting a desired blood vessel. Accordingly, the invention may provide a device having a fixation structure configured to compress the skin and underlying tissues and a detection structure comprising a sensor in the form of an ultrasonic transducer.

If the sensor of the detection structure is an ultrasonic transducer, it may be mechanically integrated to face towards the blood vessel. I.e. the transducer may be arranged in a specific direction relative to the anatomic region. The angle may have an impact on the principle of measurement, and it could be selected either for frequency shift based detection, also referred to as doppler effect, or for ultrasound depth detection being similar to the principle of a sonar.

The frequency shift based detection can detect a flow of blood passing through the blood vessel, and thereby determine if the blood vessel is an artery or a vein by detecting the direction of the flow relative to the orientation of the housing or frame. This is achievable because the orientation of the housing or frame relative to the limb is known by the computing unit, due to the restrictive fixation structure. The position of the ultrasonic transducer can then reveal the position of the intended blood vessel relative to the skin.

The device may comprise one or more sensors. These may be arranged in the housing or frame such that detection of blood flow from one or more of subset(s) of the total number of sensors reveals the position of the intended blood vessel relative to the skin.

In addition to said pressure wave detection, the sensor could be based on electromagnetic waves, e.g. near infrared spectroscopy, on radiographic detection, e.g. alpha, beta, or gamma radiation, or X-ray detection with or without contrast medium injection into the blood enabling radiographic detection as compared to the rest of the tissues.

The detection path is defined by the sensor, inter alia by its location relative to the fixation of the housing or frame to the anatomical region. The detection path could be defined e.g. by a linear or curved array of sensors, or a matrix of sensors.

The detection path may define a location and an orientation of the sensors relative to said anatomical region.

Alternatively, or additionally, the path could be defined by a movement structure, e.g. manually or motorized to enable movement of one or more sensors thereby enabling the sensors to obtain multiple positions along the detection path.

The detection structure comprises a computing unit e.g. comprising a CPU. This CPU may e.g. be attached to a printed circuit board (PCB). The sensor may be attached to the same PCB such that the detection structure is essentially constituted by a PCB with a CPU, and storage with a program enabling the CPU to define the entry location and to communicate via the interface.

The computing unit is configured to receive the signal from the sensor(s). This may be a wireless or a physical connection between the computing unit and the sensors, e.g. a cabled connection, e.g. by fiber optics or electrical cables, or they could be connected by a common PCB.

The computing unit may be attached to or form part of each of the sensor(s), or it may be separate from the sensor(s).

The computing unit may include memory means, e.g. in the form of a flash memory or similar computer memory containing re-definable data or fixed data. The memory means may include a predefined definition of the electrical signal as a response to the signal received from the sensor. This provides a safety feature since the computing unit will only react on realistic values from the sensor and disregard obviously erroneous signals.

The computing unit is configured to determine an insertion procedure including defining an entry location relative to the blood vessel for insertion of a needle or cannula into the blood vessel. This may include a point or area on the skin, an angle for the needle or cannula, and/or an insertion depth.

Further, the computing unit may comprise an interface configured to communicate the entry location to a human operator or an interface to communicate the entrance location to an insertion structure for automatic insertion of the percutaneous needle or cannula.

The communication to a human operator could include moving a guidance member indicating the entry location and optionally an angle and allowing the user to insert the needle or cannula in a guided manner.

The insertion structure could form part of the device. Such an insertion structure may include a second motorized movement structure for moving the needle and/or cannula into the patient.

The interface to a human operator may include visual, audible, or tactile interface structure(s). This may include a screen providing visual instructions, a diode or an array of diodes, a speaker, a vibration motor, or any similar structures capable of interfacing with a human operator and thereby defining the insertion procedure. The interface structures may signal one or more applicable insertion procedures, e.g. multiple diodes indicating by light one or more entry locations and/or angles relative to the skin.

The interface for a movement structure could include a data interface of any known kind, e.g. communicating data in accordance with existing data exchange standards such as RS232.

Said interface to the movement structure may incorporate said interface to a human operator, e.g. signaling when the human operator has reached an intended destination of the movement structure thereby defining the insertion procedure.

Said movement structure may be motorized and the interface may communicate control signals between the computing unit and the movement structure, e.g. controlling actuation of the movement structure, e.g. by transmitting a number of steps for a stepper motor, necessary to reach an intended destination of the movement structure thereby defining the insertion procedure.

The at least one sensor may comprise at least one receiver defining a sensing direction and configured to receive an output signal from the anatomical region. The receiver may have the structure of a microphone, and the sensing direction may be provided by the orientation of the microphone. The at least one receiver may be an ultrasonic transducer. The at least one receiver may be an electromagnetic sensor could be based on electromagnetic waves, e.g. a near infrared spectrophotometer.

The at least one sensor may comprise at least one emitter capable of emitting an input signal into the patient. This at least one sensor may be an ultrasonic transducer sending ultrasound in the direction of the patient. The at least one sensor may be comprised by an Near infrared (NIR) spectroscopy optical device based on the absorption of electromagnetic (EM) radiation at wavelengths in the range 780 to 2,500 nm. The light interacts with the sample and the sensor may configured to measure its transmittance and absorbance.

The receiver may be configured to detect the output signal in the form of an echo of the input signal emitted by the emitter.

The computing unit may be configured to determine a change in time from emission of input signal to detection of the output signal and to use the change in time to detect the location of the blood vessel. The computing unit may be configured to instruct the emitter to emit ultrasonic waves in the direction of the patient and detect the returned echoes from the tissues and from the intended blood vessel. The computing unit is configured to receive and detect the signals in the principle of sonar detecting the returned echoes.

The computing unit may be configured to determine a change in frequency between the input signal emitted by the signal emitter, said signal emitter may emit ultrasonic waves, and the output signal received by the receiver, said receiver may be configured to receive ultrasonic waves and to detect the location of the blood vessel based on frequency shift of the signal determined by the computing unit, based on the detected change in frequency.

The computing unit may be configured to use the previously described detection configurations to provide a blood flow measure indicating a blood flow in the blood vessel, which thereafter may be able to detect the presence of a blood vessel in the measured object of the device. Said computing unit may be configured to compare the measured flow and use reference data in determining whether the measured flow is a blood vessel, whether it is indicative of being an arterial or a venous blood vessel.

The computing unit may be configured to determine the blood flow measure based on the frequency shift of the signal, said computing unit may be configured to communicate with an emitter emitting ultrasound waves and configured to communicate with a receiver receiving ultrasound waves, and configured to compare the data from the emitted waves with the data from the received waves. The frequency shift may be, but is not limited to a change in frequency of a continuous waves or a change in frequency of pulsed waves.

The detection structure of the device may configured to provide a depth measure indicating a depth of the intended blood vessel below the outer skin surface. The detection structure may be configured to use the echo return time of emitted ultrasound waves, from emission to receiving the corresponding ultrasound waves, to determine the presence of an underlaying structure. Said detection structure may be configured to use the known speed of wave propagation to thereby determine the actual depth. The detection structure may also be configured to use the spatial sensitivity of Near infrared (NIR) spectroscopy to determine the depth of the intended blood vessel. The detection structure may also be configured to detect X-ray, said X-ray may be emitted by the emitter, or said X-ray may be detected by X-ray signals originating internally from the intended blood vessel following intravenous and/or intraarterial contrast medium injection by a healthcare provider.

The detection structure may be configured to provide the depth measure and/or the blood flow measure by exiting the emitter in accordance with a first principle of operation or a second principle of operation.

The at least one sensor may comprise a probe. Herein, the term "probe" may refer to any kind of transducer. The sensor may be configured to detect flow. In that case, we refer to the sensor as a flow sensing probe. Such a sensor may detect the blood flow measure or detection and the at least one sensor may comprise a separate sensor for providing the depth measure.

The flow sensing probe may be configured to define a first measuring axis along which it emits and receives signals, the depth probe may be configured to define a second measuring axis along which it emits and receives signals, and wherein the flow sensing probe and the depth probe may be arranged such that the first and second measuring axes intersect below the skin surface.

The computing unit may be configured to compare the blood flow measure and the depth measure to verify a location of an intended blood vessel. The computing unit may be configured to compare the data communicated from the emitter and/or the receiver with data recorded in memory to determine whether the depth is indicative of the intended blood vessel or whether the flow is indicative of the intended blood vessel.

The device may further comprise a carriage, which holds the at least one sensor and which is configured to be moved along the detection path. The detection path may be a medial-to-lateral anatomical line along the skin of the frontal part of a thigh, frontal part of a pelvis, frontal part of a groin, a wrist, a frontal part of an arm and/or a frontal part of a neck, said medical-to-lateral line(s) in said anatomical region(s) having large blood vessels present that cross below the skin of the detection path.

The movement structure of the device may be a first motorized movement structure arranged to move the carriage along the detection path. A such motorized movement structure is shown in the figures and explained below in the detailed description of embodiments of the invention.

The at least one sensor may be configured to provide the signal indicative of the presence of the blood vessel while moved by the carriage along the detection path. A such carriage and its structure being configured for movement is shown in the figures and explained below in the detailed description of embodiments of the invention.

The device may comprise a guidance member arranged to define a limiting passageway for manual insertion of the percutaneous needle and/or cannula through the guidance member by an operator, the limiting passageway defining an insertion direction of the percutaneous needle and/or cannula. A such guidance member and limiting pathway are shown in the figures and explained below in the detailed description of embodiments of the invention.

The device may comprise a depth limiter configured to interact with the guidance member and mechanically limit an insertion depth of the percutaneous needle and/or cannula when manually inserted through the passageway. A such depth limiter structure is shown with reference to the figures and described in the detailed description of embodiments of the invention.

The depth limiter may be configured to interact with a predefined part of the percutaneous needle and/or cannula to prevent that predefined part from passing through the passageway. A such predefined structural limitation establishing a mechanical relationship between a needle and the depth limiter is shown in the figures and explained below in the detailed description of embodiments of the invention.

The guidance member may form a part of the carriage, wherein the guidance member is structurally configured to be physically attached or be arranged into the structure of the movable carriage, such that when the carriage is moved either manually or automatically, so is the guidance member accordingly. A such carriage may be configured as shown in the detailed description of embodiments of the invention.

The insertion structure for automatic insertion of the percutaneous needle and/or cannula may comprise a second motorized movement structure configured for moving the percutaneous needle and/or cannula and/or the guidance member relative to the housing or frame. A such second motorized movement structure is shown in the figures and explained below in the detailed description of embodiments of the invention.

The second motorized movement structure is configured to introduce the percutaneous needle and/or cannula into the anatomical region into the blood vessel. A such second motorized may be configured to form a part of the carriage, wherein the second motorized movement structure moves the guidance member, with or without a needle and/or cannula already present in the guidance member, according to the position of the carriage itself. Thereby the second motorized movement structure may facilitate the movement of the guidance member and/or needle and/or cannula without moving the carriage along the detection path, i.e. without moving the location of the carriage in relation to the skin. The second motorized movement means may also be configured to facilitate movement simultaneously with the activity of the first motorized movement structure.

The above motorized movement structures may be configured as shown in the detailed description of embodiments of the invention.

Said second motorized movement structure may be configured to receive a signal from the computing unit (defining e.g. a number of steps for a stepper motor. Said stepper motor may be a part of or comprise the first and/or second motorized movement structures. Said motorized movement structures may also be comprised by other mechanical actuators in the known art).

The depth limiter may be comprised of an insertion stopper. A such insertion stopper may be configured as shown in the figures and explained below in the detailed description of embodiments of the invention.

The insertion stopper may be a mechanical insertion stopper arranged to be moved by the second motorized movement structure. A such insertion stopper may be configures as shown in the figures and explained below in the detailed description of embodiments of the invention.

The depth limiter may be electronically controlled to engage upon vessel puncture detection. Such a depth limiter may be limited by the activation of a shutter mechanism, such as in e.g. a camera, that is activated by a signal from the computing unit.

The puncture detection may be configured to be performed by one, or a combination, of:
- detection of a change in insertion resistive force on the percutaneous needle and/or cannula;
- detection of blood pressure through a lumen of the percutaneous needle and/or cannula; and/or
- detection of blood passing through a lumen of the percutaneous needle and/or cannula.
- detection of change in electrical impedance measured from the tip of the needle to another point electrically connected to the subject being examined, e.g. the skin.

The blood may be detected by a sensor selected from a group consisting of, but not limited to, a photo sensor, an electrical impedance sensor and pressure sensor.

The computing unit may be configured to define an insertion depth based on the depth measure and to communicate the insertion depth to the interface.

The detection structure may be comprised of a plurality of sensors arranged along the detection path to provide signals indicative of the presence of a blood vessel and a computing unit receiving signals from the sensors. Such a plurality of sensors arranged along the detection path is shown in the figures and explained in the detailed description of embodiments of the invention.

The guidance member(s) may be arranged to define a limiting passageway for each of the plurality of sensors. A such plurality of guidance members along the detection path is shown in the figures and explained below in the detailed description of embodiments of the invention.

The plurality of sensors and the guidance members may be configured to be fixed in a mutual connection.

The fixation structure for fixation of the housing or frame to a skin surface of the anatomical region of the living being may be configured to arrange the plurality of sensors and the guidance members in a fixed position relative to the anatomical region.

In a second aspect, the invention provides a method of inserting a percutaneous object, e.g. a needle, cannula, catheter, pump and/or another interventional vascular medical device, into a blood vessel, by way of use of the herein described embodiments of device(s), the method may be comprised arranging the contact area against an outer skin surface in an orientation across an underlying blood vessel, and receiving a signal from an interface formed as part of said embodiments of device(s) for detecting the presence of a blood vessel.

Accordingly, the embodiments enable the delivery of particularly life-saving minimally invasive treatments, where the delivery of said blood vessel access is not possible today, either due to time constraints, lack of available of the correct type of highly trained specialty personnel, e.g. at most hospitals and in the out-of-hospital emergency care setting, manual procedural difficulties in performing the procedure by the operator, lack of experience and resources by the presently available medical personnel, who may not have the necessary volume of experience or procedure competency to perform the required vascular access procedure.

Since the computing unit communicates the entry location to an interface, the device can facilitate emergency blood vessel access in highly time critical emergency situations, or elective circumstances that present unusual clinical difficulties, and can facilitate treatment where it is presently impossible.

Another embodiment of the device may be configured to facilitate the steps required for the blood vessel access procedure up until the insertion of the needle into the blood vessel, whereafter the user solely inserts the needle through a proximal opening of said device. This may e.g. be enabled by moving a blood vessel access guidance member, configured with a proximal opening and distal opening, through said member a lumen connects said proximal and distal openings, said openings being configured to enable the passage of a blood vessel access needle. In one embodiment the device may mechanically position said blood vessel access member to a position on the patient, which, by the device processing means has been determined as the position with highest probability of success for attaining blood vessel access. Both the needle and the blood vessel access guidance member may be configured to ensure a specific needle insertion depth through the blood vessel access guidance member. This may be attained through a mechanical relationship between the needle and the guidance member, which limits the insertable depth of the needle through the guidance member, e.g. via a mechanical obstruction that only allows for a partial insertion of the needle or other mechanical means, or via an auditory or visual or tactile alarm activated by the device once the needle has reached or exceeded a specific needle insertion depth, said alarm may be activated by e.g. magnetic detection the needle depth, RFID or other detection means fixed place as well as limiting the depth to which it can be inserted.

The user applying a force on the proximal part of the needle towards the patient through the blood vessel access guidance member, after the device has positioned said guidance member above the intended anatomical location, will thereby facilitate a successful blood vessel access.

Large blood vessels pass through the limbs (i.e. the arms and legs) and the neck, stretching from the proximal to the distal part of the limb or the neck. The large blood vessels can be described to pass through the transverse (horizontal) plane at an angle approximately perpendicular to the plane. Scanning the transverse plane through a sensor will thus reveal the large blood vessels at their respective points of intersection with the plane. A transverse plane through the limb or the neck of the patient will thus have a point of intersection with a large blood vessel, regardless of the proximity or distality.

A sensor with perpendicular scanning direction to the first motorized movement means, will complete a scan of the plane contained within the two axes. The device may be mechanically configured to ensure that this scanned plane is the transverse plane, and thereby also that the intended blood vessel is within the section of the scan.

The sensor may comprise a signal emitter and an ability to detect a received signal, wherein a received signal may refer to a signal emitted from a transducer which is returned to the sensor through scattering, reflection, or emission. A received signal may refer to a part of the magnetic resonance imaging process. In one embodiment no signal is emitted by a transducer, and the received signal may originate in the intended blood vessel, such as sound waves produced by turbulence.

In one embodiment, the sensor may be a depth sensor (i.e. ultrasound depth sensor) capable of emitting a signal and detecting its echo. Algorithms may then recognize discrepancies in the time for the echo to return and use this to instruct a first and/or second motorized movement means. The computing means will then also use said echo return time to determine distance to the blood vessel, which can be considered the vessels coordinate along the sensing axis. Coordinate data of the vessel along the sensing axis and the location of the sensor along the axis of sideways (lateral) movement will produce a two-dimensional coordinate of the blood vessel's location on the transverse plane.

One embodiment of the device may include a bilateral carriage. Said carriage may contain both the sensor as well as a percutaneous needle or cannula or guidance member. The carriage may be moved by the first motorized movement means, such that the sensor and other components on the carriage are always at the same location on the patient.

The computing means in one embodiment may be configured to utilize said transverse plane coordinate to instruct a second motorized movement means, for insertion of a percutaneous cannula or needle.

One embodiment of the device may include a sensor, wherein data from the sensor is sent to the computing unit, which determines the direction of the blood flow. The computations unit determines the direction of blood flow, which can signal the interface and distinguish between an artery and a vein, or several arteries and veins. This is due to the directionality of blood flow in veins versus arteries, where the blood flow in veins flows toward the heart whereas the blood flow in arteries flows away from the heart.

The ability of the device to detect and locate large blood vessels may also be applied to achieve guidance or insertion in(to) tissue(s) that are determined to not be blood vessel(s). One embodiment of the device may use the determined relative presences of large blood vessels in order to identify the location of said blood vessels, and specifically communicate to the interface where the blood vessels are not present, to thus facilitate e.g., intramuscular locations, where a puncture into tissues that are not blood vessels are intended. Said locations may facilitate injections of e.g. vaccinations or other intramuscular injections, either through guidance of an operator via the interface in an embodiment of the device, or via motorized movement means in another embodiment of the device.

### LIST OF NUMBERED EMBODIMENTS

1. A blood vessel detection device comprising:
   - a housing or frame;
   - fixation structure for fixation of the housing or frame to a skin surface of an anatomical region of a living being, the anatomical region selected from a group consisting of: frontal part of a thigh, frontal part of a pelvis, frontal part of a groin, a wrist, a frontal part of an arm and a frontal part of a neck, and
   - a detection structure for detecting presence of a blood vessel, the detection structure defining a detection path along the skin surface, and comprising:
      ∘ at least one sensor arranged to provide a signal indicative of the presence of a blood vessel along the detection path;
      ∘ a computing unit configured to:
         receive the signal; and to define an insertion procedure defining an entry location on the skin surface where a percutaneous needle and/or cannula can be inserted into the blood vessel,
      wherein the computing unit comprises an interface configured to communicate the entry location to a user for manual insertion or to an insertion structure for automatic insertion of the percutaneous needle and/or cannula.
2. The device according to embodiment 1, comprising the insertion structure for automatic insertion for the percutaneous needle and/or cannula.
3. The device according to embodiment 1, wherein the at least one sensor comprises at least one receiver configured to receive an output signal from the anatomical region.
4. The device according to any of the preceding embodiments, wherein the at least one sensor comprises at least one emitter capable of emitting an input signal into the patient.
5. The device according to embodiment 4, wherein the sensor is configured to detect the output signal in the form of an echo of the input signal emitted by the emitter.
6. The device according to embodiment 5, wherein the computing unit is configured to determine an echo return time, from emission of input signal to detection of the output signal and to use the change in time to detect the location of the blood vessel.
7. The device according to embodiments 3 and 4, wherein the computing unit is configured to determine a change in frequency between the input signal emitted by the signal emitter and the output signal received by the sensor, and to detect the location of the blood vessel based on the change in frequency.
8. The device according to any of the preceding embodiments, wherein the computing unit is configured to a provide a blood flow measure or recognition, indicating a blood flow in the blood vessel.
9. The device according to embodiment 7 and 8, wherein the computing unit is configured to determine the blood flow direction based on the frequency shift of the output signal relative to the input signal.
10. The device according to any of the preceding embodiments, wherein the detection structure is configured to provide a depth measure indicating a depth of said blood vessel below the outer skin surface.
11. The device according to embodiments 4 and 8 or according to embodiment 4 and 10, wherein the detection structure is configured to provide the depth measure and/or the blood flow measure by exiting the emitter in accordance with a first principle of operation or a second principle of operation.
12. The device according to embodiments 8 and 10, wherein the at least one sensor comprises a flow sensing probe for providing the blood flow measure and a separate depth probe for providing the depth measure.
13. The device according to embodiment 12, wherein the flow sensing probe defines a first measuring axis along which it emits and receives signals, the depth probe defines a second measuring axis along which it emits and receives signals, and wherein the flow sensing probe and the depth probe are arranged such that the first and second measuring axes intersect below the skin surface.
14. The device according to embodiments 8 and 10, wherein the computing unit is configured to compare the blood flow measure and the depth measure to verify a location of a blood vessel.
15. The device according to any of the preceding embodiments, comprising a carriage which holds the at least one sensor and which is configured to be moved along the detection path.
16. The device according to embodiment 15, comprising a first motorized movement structure arranged to move the carriage along the detection path.
17. The device according to embodiment 15 or 16, wherein the at least one sensor is configured to provide the signal indicative of the presence of the blood vessel while moved by the carriage along the detection path.
18. The device according to any of the preceding embodiments, comprising a guidance member allowing guided manual insertion of the percutaneous needle and/or cannula into the entry location.
19. The device according to embodiment 18, wherein the guidance member is arranged to define a limiting passageway for manual insertion of the percutaneous needle and/or cannula through the guidance member by an operator, the limiting passageway defining an insertion direction of the percutaneous needle and/or cannula.
20. The device according to embodiment 19, comprising a depth limiter configured to mechanically limit an insertion depth of the percutaneous needle and/or cannula when manually inserted through the passageway.
21. The device according to embodiment 20, wherein the depth limiter is configured to interface with a predefined part of the percutaneous needle and/or cannula to prevent that predefined part from passing through the passageway.
22. The device according to any of embodiments 15-17 and 18-21, wherein the guidance member forms part of the carriage.
23. The device according to any of the preceding embodiments, further comprising a second motorized movement structure configured for moving the percutaneous needle and/or cannula and/or the guidance member relative to the housing or frame.
24. The device according to embodiment 2 and 23, wherein the second motorized movement structure forms part of the insertion structure for automatic insertion of the percutaneous needle or cannula.
25. The device according to embodiment 23 or 24, wherein the second motorized movement structure is configured to introduce the percutaneous needle and/or cannula into the anatomical region into the blood vessel.
26. The device according to embodiment 23 or 25, wherein said second motorized movement structure is configured to receive a signal from the computing unit.
27. The device according to embodiment 20 , wherein the depth limiter comprises an insertion stopper.
28. The device according to embodiment 27, wherein the insertion stopper is a mechanical insertion stopper arranged to be moved by the second motorized movement structure.
29. The device according to embodiment 20-28, wherein the depth limiter is electronically controlled to engage upon vessel puncture detection.
30. The device according to embodiment 29, wherein puncture detection is performed by one of:
   - detection of change in insertion resistive force on the percutaneous needle and/or cannula;
   - detection of characteristic waveform of normal force asserted by the vessel wall to the needle and/or cannula upon the moment of puncture.
   - detection of blood pressure through a lumen of the percutaneous needle and/or cannula; and
   - detection of blood passing through a lumen of the percutaneous needle and/or cannula.
   - detection of characteristic change in electrical impedance from the tip of the needle and/or cannula relative another electrical junction on the patient being punctured, said junction could be incorporated within the needle/cannula or anywhere within the housing/frame touching the patient's skin.
31. The device according to embodiment 10, wherein the computing unit is configured to define an insertion depth based on the depth measure and to communicate the insertion depth to the interface.
32. The device according to any of the preceding embodiments, wherein the detection structure comprises a plurality of sensors arranged along the detection path to provide signals indicative of the presence of a blood vessel.
33. The device according to embodiment 32, comprising a guidance member arranged to define a limiting passageway for each of the plurality of sensors.
34. The device according to embodiment 32 and 33, wherein the plurality of sensors and the guidance members are in fixed mutual connection.
35. The device according to any of embodiments 32-34, wherein the fixation means for fixation of the housing or frame to a skin surface of the anatomical region of the living being is configured to arrange the plurality of sensors and the guidance members in a fixed position relative to the anatomical region.
36. A method of inserting a percutaneous object into a blood vessel by use of a device according to any of embodiments 1-35, the method comprising arranging the contact area against an outer skin surface in an orientation laterally across an underlying blood vessel, and actuating the detection structure for detecting presence of the blood vessel.

### BRIEF DESCRIPTION OF DRAWINGS

Figure 1 illustrates a perspective of an embodiment of the invention with two ultrasonic transducers: one arranged for flow detection, which makes contact with the anatomical region at ca. 60° allowing it to detect blood flow; an ultrasonic transducer configured ideally for depth detection, perpendicular to the skin of the patient, able to send an ultrasonic pulse and detect the echoes.
Figure 2 illustrates another perspective of the embodiment in figure 1.
Figure 3 illustrates an up-close view of the transducers and other movable components featured in figure 1 and 2.
Figure 4 illustrates an embodiment of the casing and fixation structure of the device.
Figure 5 illustrates an embodiment of the casing and fixation structure of the device.
Figure 6 illustrates a rendering of the embodiment in figure 1, placed on the upper left groin of the patient, thereby enabling access to the femoral artery.
Figure 7 illustrates a rendering of the embodiment in figure 4.
Figure 8 illustrates an embodiment of an insertion stopper.
Figure 9 illustrates a 3D rendering of the device in figure 8.
Figure 10 illustrates components from an embodiment with autonomous detection and manual insertion.
Figure 11 illustrates an embodiment similar to figure 10, with a housing and belt structure.
Figure 12 illustrates a cross section view of the device, patient's thigh and desired blood vessel.
Figure 13 illustrates a rendering of select components from an embodiment of the device.
Figure 14 illustrates a rendering of select components from an embodiment of the device.
Figure 15 illustrates a rendering of select components from an embodiment of the device.
Figure 16 is a photograph of a functioning prototype embodiment of the device.
Figure 17 illustrates a rendering of select components from an embodiment of the device, responsible for encasing the probe and providing a cavity for ultrasound gel.
Figure 18 illustrates another rendering of the embodiment shown in figure 17.

### DETAILED DESCRIPTION OF EMBODIMENTS

In the following, the embodiments are grouped in embodiments 1-7. Each of the numbered embodiments 1-7 may be combined with other embodiments. Accordingly, the features included in one embodiment may be included also in the other embodiments without being specifically mentioned in relation to those other embodiments.

### EMBODIMENT 1

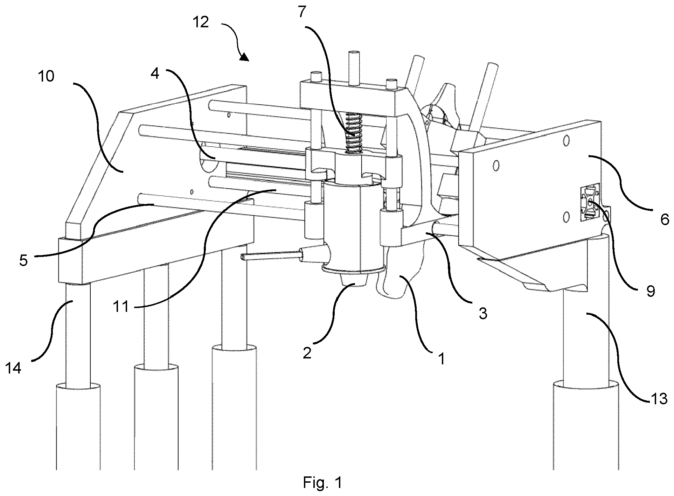

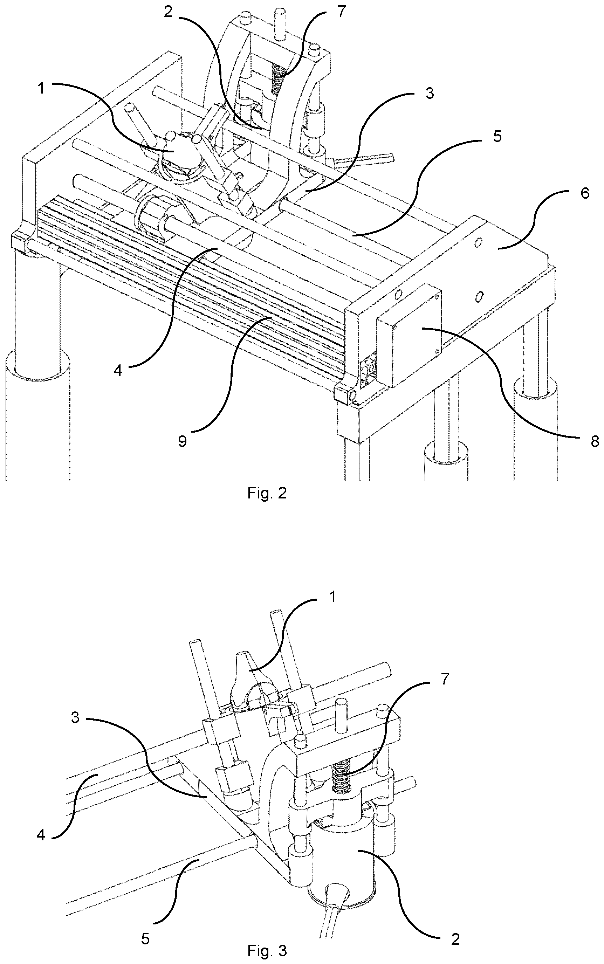

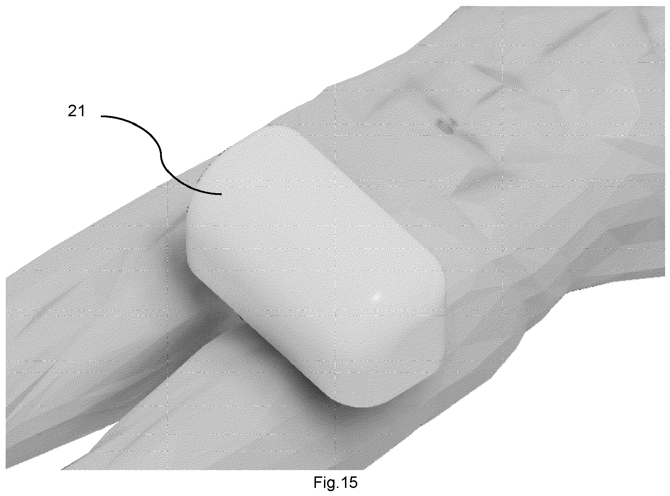

In embodiment 1, the device may include a movable carriage forming the detection path, and it may include a number of sub embodiment of the invention shown in figures 1, 2, 3, 6, 12, 13, 14, 15, 16, and 17.

### FIGURE LEGENDS:

A sensor in the form of a doppler probe **1**
A sensor in the form of a depth probe **2**
Component carriage **3**
Drive shaft **4**
Proximal carriage rail **5** defining a detection path
Medial side plate **6**
Probe spring **7**
Motor **8**
Square support beam **9**
Lateral side plate **10**
Distal carriage rail **11**
Patient mountable device **12**
Medial support pillar **13**
Lateral support pillars **14**
Femoral artery **39**
Doppler probe sensing direction **40**
Sensing direction of depth sensor **41**
Atraumatic depth probe case **42**
A frame **46**

Fig. 1 illustrates the patient mountable device **12.** The device **12** is contained within a housing formed as a frame, defined by the medial side plate **6** and the lateral side plate **10.**

The device in this embodiment includes a sensor in the form of two transducers, an ultrasound doppler transducer **1** and an ultrasound depth transducer **2.** Other embodiments may utilize different detection techniques and/or transducer configurations. In the shown embodiment, the doppler transducer **1** facilitates flow detection. As described, the flow detection allows the device to distinguish between arteries and veins, due to its restricted attachment to a limb and the computing unit's physiological knowledge of blood flow in blood vessels through a limb.

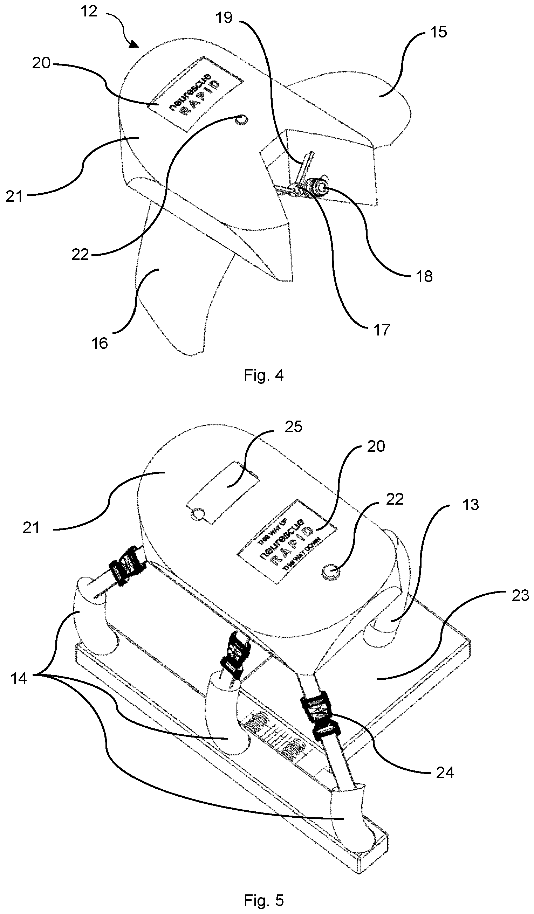

The device **12** is configured to be mounted onto a patient's limb, such that the direction of the motorized movement means, powered by the motor **8,** intersects the intended blood vessel. Other embodiments of the invention may be compatible with limbs or anatomical regions on the either side of the line of symmetry or sagittal plane. In said embodiment, the device could be moved to the contralateral side of the patient, if insertion fails on the first side of where the device was initially attached, and where a procedure was attempted first. The electrical stepper motor **8,** turns the drive shaft **4,** as instructed by the computing unit, moving the component carriage **3** (and thus also the contained doppler probe **1,** depth probe **2** and probe spring **7)** along the proximal carriage rail **5** and distal carriage rail **11** and across the patient's limb. The attachment in this embodiment is done using pillars (medial support pillar **13** and lateral support pillars **14).** Said pillars may be connected to a base plate (a base plate **23** is shown in fig. 5) below the limb, thus firmly pushing the device against the surface of the patient through a pulling force between base plate and side plates **(6** and **10),** said pulling force being required for establishing adequate contact between the transducers and the skin of the anatomical region. The forces induced on the housing, defined by the medial side plate **6** and lateral side plate **11,** require rigidity of the structure; strength will be enhanced by the application of load bearing structures such as the square supporting beam **9.** Other embodiments may incorporate more of similar structures, to further increase rigidity. Moreover, said square supporting beam **9,** also provides a surface that can be firmly pushed against the skin, when attaching the device **12,** without hindering the motion of moving components, such as the component carriage **3.**

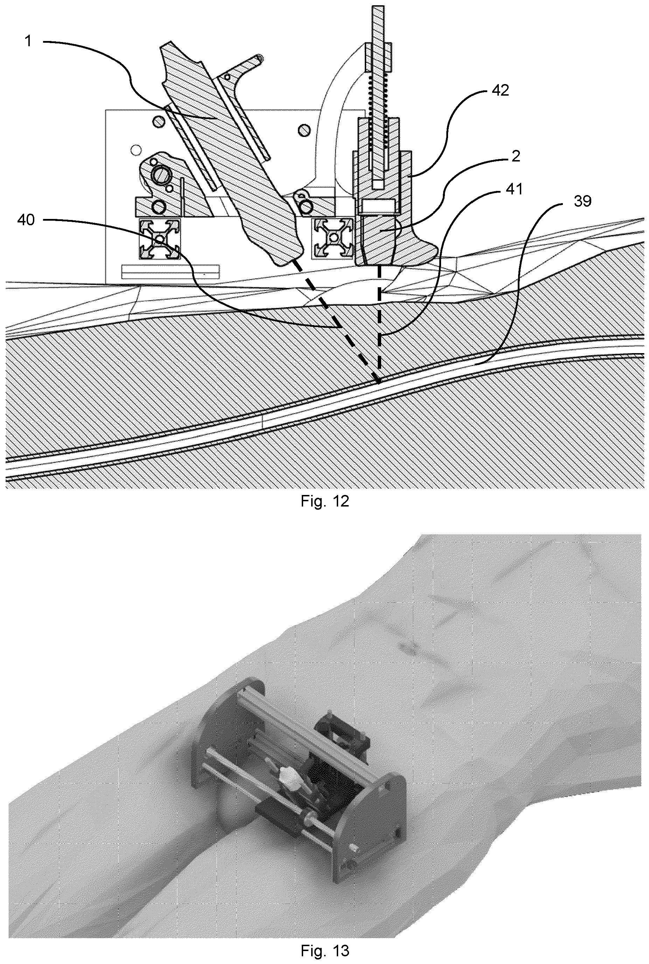

Figure 12 shows a cross section view of the device and the patient such that the intended blood vessel is visible. In figure 12, the dimensions of and the shape of the housing and frame and fixations means are intended for the femoral artery as the intended blood vessel **39.** Another vessel may be intended by other embodiments. Figure 12 also communicates the general depth transducer sensing direction **40** and the doppler transducer sensing direction **41.** The general sensing direction is not a physical component but a line showing the orientation of the doppler probe 1 and the depth probe **2.** The transducers may receive sensing data from multiple different directions simultaneously, but the general depth probe sensing direction **40** and the doppler probe sensing direction **41** show the axis around which the sensing is centered.

Figure 12 has an embodiment which further comprises an addition not featured in the embodiments shown in figures 1, 2, 3 and 6, which is a rounded atraumatic depth transducer protector case **42.** Said component protects the patient from the sharp edge of the depth transducer **2,** as it moves across the skin of the patient. Such a transducer protector case, er emitter or detector protector case, may be included as part of all the previously mentioned embodiments.

### EMBODIMENT 2

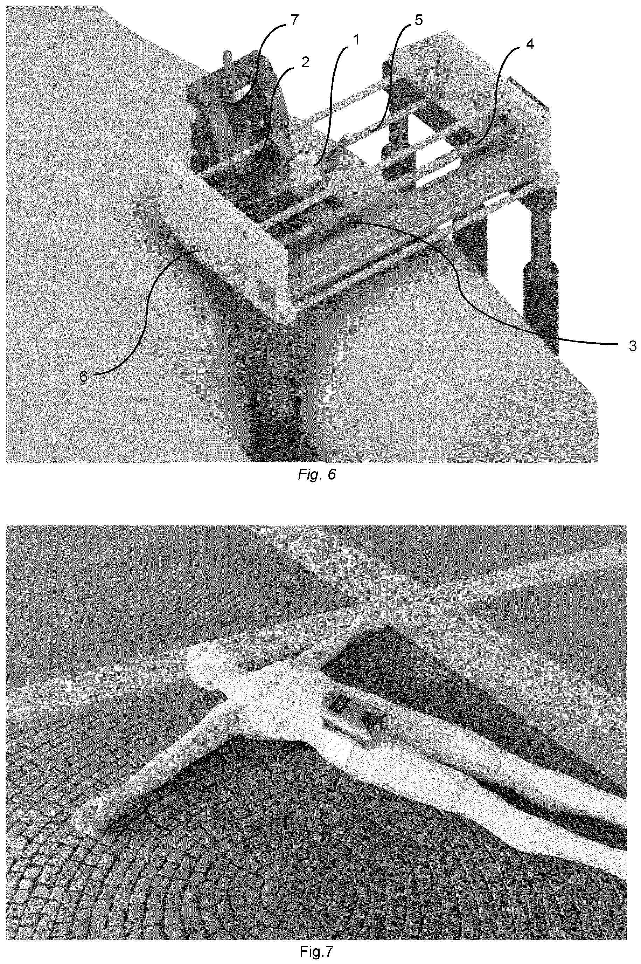

Other embodiments of the invention are shown in figures 4 and 7.

### FIGURE LEGENDS:

Patient mountable device **12**
Medial adhesive flap **15**
Lateral adhesive flap **16**
Sheath **17**
Proximal sheath opening **18**
Sheath holder **19**
Screen **20**
Housing **21**
Button **22**

In figures 4 and 7 the device **12,** includes an encapsulating housing **21,** which may contain several components described in embodiment 1. In embodiment 2, the method of attachment of the device to the surface of the skin is done by use of the medial adhesive flap **15** and the lateral adhesive flap **16.** A sheath **17** is also depicted in both figures, providing vascular access to the intended blood vessel through the proximal sheath opening **18.** The sheath **17** is held in its intended place by use of the sheath holder **19** on the device.

The device **12** may gain user input using a button **22** and provide information output to user/users through a screen **20.** In some embodiments, the procedure may require the confirmation of a user, which may be provided using said button **22;** the procedure may also be stopped by use of the same button or another button in other embodiments. The screen **20** may also be a touch screen, serving as a communication interface for both user input and output to the user.

### EMBODIMENT 3

### FIGURE LEGENDS:

Patient mountable device **12**
Medial support pillar **13**
Lateral support pillars **14**
Screen **20**
Housing **21**
Button **22**
Base plate **23**
Buckle **24**
Compartment **25**

A third embodiment of the invention is shown in figure 5. Figure 5 depicts the base plate **23** mentioned embodiment 1, but not shown in fig. 1, 2 and 3. The method of attachment thus also relies on attaching the device **12** around a limb and connecting the housing **21** to the base plate **23** using the medial support pillar and lateral support pillars **14.** In embodiment 3, the method of attachment of the housing **21** to the pillars **13** and **14** uses buckles such as buckle **24,** which can be connected by the user upon attachment. This embodiment may also further comprise a compartment **14** for other components such as ECG leads, or other biological sensors, if additional biological data is obtained.

### EMBODIMENT 4

### FIGURE LEGENDS:

Drive shaft **26**
Insertion stopper **27**
Sheath **28**
Guider **29**
Needle hub **30**
Needle **31**

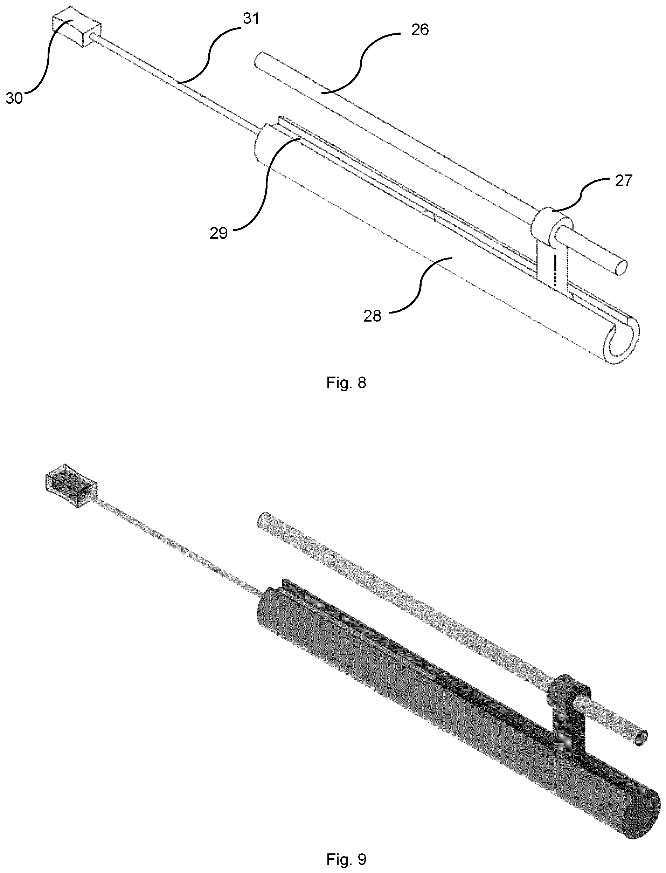

An embodiment of a feature of the invention is shown in figures 8 and 9 and said feature may be included in any of the mentioned embodiments. Said feature has a guider **26** with the lumen for the needle **31.** The guider **29** can slide within the lumen of the sheath **28** until the distal end of the guider **29** is stopped by the insertion stopper **27.** The location of the insertion stopper may be moved by the rotation of the depicted drive shaft **26,** as instructed by the computing unit. The needle hub **30** is larger than the lumen of the guider **29** and is thus also stopped once the guider **29** makes contact with the insertion stopper **27.** Puncture detection could utilize the needle hub **30,** as blood from the intended vessel is allowed to flow through a needle lumen to the hub, which may be recognized by sensors with blood contact detecting features as known in the state of the art. The embodiment of this feature will prevent the needle from passing through the backside of the intended blood vessel, ensuring that the distal end of the needle does not pierce completely through the intended blood vessel due to too deep an insertion having been performed.

### EMBODIMENT 5

### FIGURE LEGENDS:

Housing **21**
Transducer **32**
Guidance member **33**
Array of transducers **34**
Indicator **35**
Array of guidance members **36**
Abdominal fixation structure **37**
Thigh fixation structure **38**

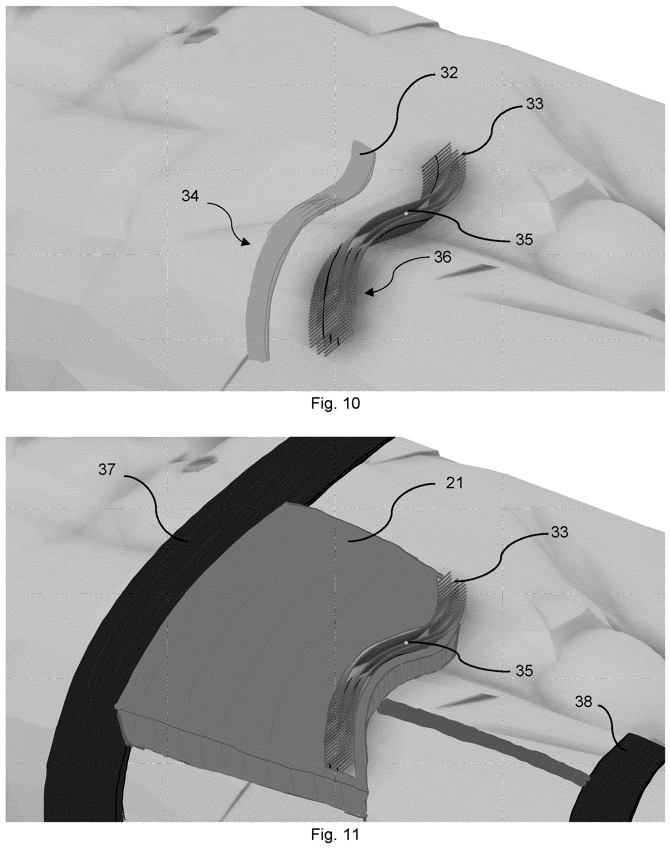

Another embodiment is shown in figures 10 and 11. In the shown embodiment, the need for moving components is minimized by an array of duplicated components; namely the array of transducers **34,** which comprises a series of components, such as transducer(s) **32,** arranged along the limb of the patient, such that the array sensing direction intersects with the intended blood vessel. Similar to the array of transducers **34,** an array of guidance members **36** comprises a series of duplications of the individual guidance members, e.g. needle openings **33** facing e.g. a pre-determined direction and angle, e.g. facing ca. 45 degrees towards the skin surface, such that the direction of the array of guidance members **36,** intersect with the intended blood vessel.

During the intended use of the embodiment, the computing unit may determine the ideal insertion location based on data from the array of transducers **36.** The data from the transducers may be compared to see where along the array the intended blood vessel is present or likely to be present. The computing unit may then communicate with an interface to instruct the user which guidance member to use (e.g. insert a needle through) to gain access to the intended blood vessel through. Said communication interface may be embodied using e.g. an LED indicator **35,** wherein one of several LED indicators lights up, as shown in the figure. This embodiment may further include the features outlined in EMBODIMENT 4', to limit the depth of insertion, but a simple lumen with no insertion stopper may also be incorporated.

Figure 11 shows how the embodiment of the device, partially encased in a housing **21** configured to attached to the patient using an abdominal fixation structure **37** and a thigh fixation structure **38.** Said fixation structures may be realized using any variety of the previously mentioned fixation structure described in the previous embodiments.

### EMBODIMENT 6

In this embodiment, the fixation structure is not constituted by a belt or strap.
Housing **21**
Patient mountable device **12**

Another embodiment of the invention is shown in figure 13, 14 and 15. The embodiment may feature components previously discussed, such as loop fasteners with Velcro^{®} or adhesive straps, as a means of fixing the patient mountable device **12** to the surface of the patient. In said figures the device **12** is attached using the friction between the surface of the device **12** and the patient. The friction force fixes the device **12** to the skin of the patient and depends on the normal force and the coefficient of friction of the two interfacing surfaces. In this embodiment, the friction surface constitutes a part of the fixation structure.

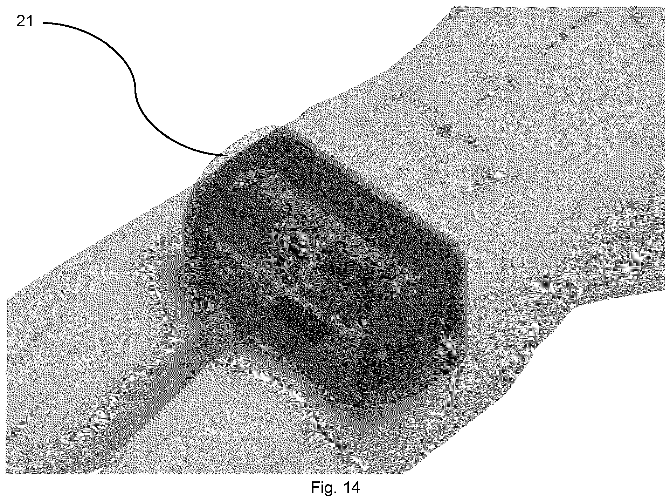

The device **12** may thus be fitted with a material with a high coefficient of friction on the surface intended to be in contact with the skin. During use of the device, a force may be applied on the device towards the patient by the user, to further increase the friction force. To increase the force pushing the device towards the patient, suction may be used by creating low pressure in the housing **21** (housing shown on figure 13 and 14). Such means for creating low pressure may also be considered to constitute a part of the fixation structure.

The housing **21** in figure 13 and 14, may further act as a component able to restrict placement, to increase probability of correct placement above the desired blood vessel. In figure 13 the housing **21** is depicted as transparent while it is depicted in a non-transparent material in figure 14. The housing **21** is shaped in such a way that only the desired surface is compatible with the curvature of the desired anatomical region, thus restricting the orientation around one axis and increasing the probability of successful detection of the desired vessel.

### EMBODIMENT 7

Syringe **43**
Ultrasound gel lumen **44**
Ultrasound gel cavity **45**

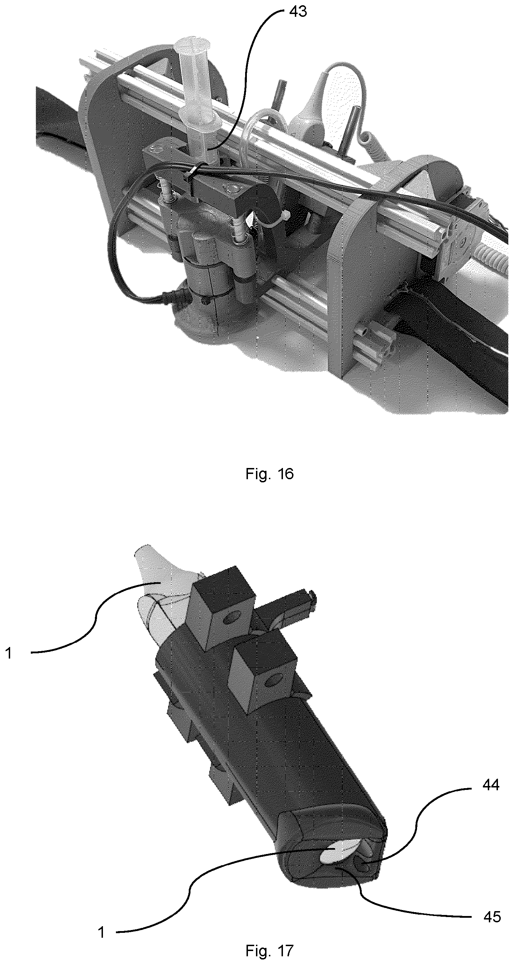

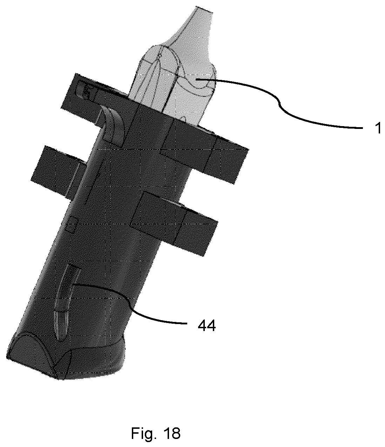

An embodiment of the device incorporating many of the already mentioned features is shown in figure 16. The embodiment has been realized and built as shown if the photograph in figure 17. The fixation means utilized in the embodiment is loop fasteners with Velcro^{®} (which extend beyond the image). A syringe **43** containing ultrasound gel has been included. Said syringe **43** is connected by a lumen **44** to a cavity **45** surrounding the probe head (figure 17 and 18 do not include the syringe but is intended to be assembled like shown in figure 16), in order to create a seamless ultrasound medium, regardless of varying curvature along the skin of the patient.

### EXAMPLE 1

Operation of an embodiment of the device: The device is placed on a patient's limb, in a manner determined by the construction of the frame and the device fixation structure.

An unexpectedly faulty depth sensor leads to an incorrect first indication of the intended blood vessel presence. The doppler probe will indicate another second intended blood vessel location, and a discrepancy between these two detected locations, greater than an acceptable margin, will result in an error warning from the computing unit. Furthermore, the gathered data is compared to known data about the anatomy and physiology of the anatomical region, thus for example producing an error warning, if the intended vessel presence is detected at >10 mm skin depth.

The procedure is halted, and the computing unit signals the interface, accordingly, thus preventing an erroneously performed procedure.

### EXAMPLE 2

Operation of an embodiment of the device: A transducer in the form of a doppler probe, is excited by an electrical signal to emit an ultrasonic pulse. Said ultrasonic pulse is directed into the patient, where the sound waves are reflected off internal structures. By directing the waves in a non-perpendicular direction to a movement/flow of underlying tissue (blood flow), the returned ultrasound wave(s) will be changed in a manner that can be used to calculate the rate and direction of flow within the patient.

Said methods and embodiment may allow detection and identification of the desired blood vessel. In embodiments where the femoral artery is the desired blood vessel, it will correspond the location with the largest flow in the proximal direction.

## Claims

1. A blood vessel detection device comprising:
- a housing or frame;
- fixation structure for fixation of the housing or frame to a skin surface of an anatomical region of a living being, the anatomical region selected from a group consisting of: frontal part of a thigh, frontal part of a pelvis, frontal part of a groin, a wrist, a frontal part of an arm and a frontal part of a neck, and
- a detection structure for detecting presence of a blood vessel, the detection structure defining a detection path along the skin surface, and comprising:
∘ at least one sensor arranged to provide a signal indicative of the presence of a blood vessel along the detection path;
∘ a computing unit configured to:
receive the signal; and to define an insertion procedure defining an entry location on the skin surface where a percutaneous needle and/or cannula can be inserted into the blood vessel,
wherein the computing unit comprises an interface configured to communicate the entry location to a user for manual insertion or to an insertion structure for automatic insertion of the percutaneous needle and/or cannula.

2. The device according to claim 1, comprising the insertion structure for automatic insertion for the percutaneous needle and/or cannula.

3. The device according to claim 1, wherein the at least one sensor comprises at least one receiver configured to receive an output signal from the anatomical region.

4. The device according to any of the preceding claims, wherein the at least one sensor comprises at least one emitter capable of emitting an input signal into the patient.

5. The device according to claim 4, wherein the sensor is configured to detect the output signal in the form of an echo of the input signal emitted by the emitter.

6. The device according to claim 5, wherein the computing unit is configured to determine an echo return time, from emission of input signal to detection of the output signal and to use the change in time to detect the location of the blood vessel.

7. The device according to claims 3 and 4, wherein the computing unit is configured to determine a change in frequency between the input signal emitted by the signal emitter and the output signal received by the sensor, and to detect the location of the blood vessel based on the change in frequency.

8. The device according to any of the preceding claims, wherein the computing unit is configured to a provide a blood flow measure or recognition, indicating a blood flow in the blood vessel.

9. The device according to claim 7 and 8, wherein the computing unit is configured to determine the blood flow direction based on the frequency shift of the output signal relative to the input signal.

10. The device according to any of the preceding claims, wherein the detection structure is configured to provide a depth measure indicating a depth of said blood vessel below the outer skin surface.

11. The device according to claims 4 and 8 or according to claim 4 and 10, wherein the detection structure is configured to provide the depth measure and/or the blood flow measure by exiting the emitter in accordance with a first principle of operation or a second principle of operation.

12. The device according to claims 8 and 10, wherein the at least one sensor comprises a flow sensing probe for providing the blood flow measure and a separate depth probe for providing the depth measure.

13. The device according to claim 12, wherein the flow sensing probe defines a first measuring axis along which it emits and receives signals, the depth probe defines a second measuring axis along which it emits and receives signals, and wherein the flow sensing probe and the depth probe are arranged such that the first and second measuring axes intersect below the skin surface.

14. The device according to claims 8 and 10, wherein the computing unit is configured to compare the blood flow measure and the depth measure to verify a location of a blood vessel.

15. The device according to any of the preceding claims, comprising a carriage which holds the at least one sensor and which is configured to be moved along the detection path.
